# EUROPEAN PATENT APPLICATION

(11) **EP 1 535 514 A1**
(43) Date of publication of application: **01.06.2005**
(21) Application number: 03791435.5
(22) Date of filing: 29.08.2003
(51) Int. Cl.: A01N 1/02

(54) **COMPOSITION FOR PROTECTING ORGAN, TISSUE OR CELL AND UTILIZATION THEREOF**

(30) Priority: 30.08.2002 JP 2002255979
(71) Applicant: MG Pharmacy Inc., Kyoto-shi, Kyoto 601-8023 (JP); Komeda, Masashi, Kyoto-shi, Kyoto 602-8024 (JP)
(72) Inventor: KOMEDA, Masashi, 303 Goshonishi Urbanlife, Kamigyo-ku, Kyoto-shi, Kyoto 602-8024 (JP); HYON, Suong-Hyu, Uji-shi, Kyoto 611-0021 (JP); MIWA, Senri, Nishinomiya-shi, Hyogo 662-0037 (JP)
(74) Representative: Williams, Aylsa
(86) International application number: PCT/JP2003/011127
(87) International publication number: WO 2004/019680

(57) **Abstract**

The present invention provides a composition for efficiently protecting and preserving organs, tissues or cells. In particular, the present invention provides a protective composition which can be used during operations. Specifically, the present invention provides a composition for protecting and preserving organs, tissues or cells, comprising a polyphenol. A method is also provided for protecting an organ, tissue or cell in a subject, comprising 1) exposing the organ, tissue or cell to a polyphenol. Such a composition and a polyphenol is effective in protecting a function of an organ (particularly, the heart, brain, nerve, spinal cord and the like).

## Description

### FIELD OF THE INVENTION

The present invention relates to the protection and preservation of cells, tissues, or organs. More particularly, the present invention relates to a composition capable of protecting and preserving organs, cells, and tissue, and the use of the same.

### BACKGROUND ART

Usually, cells are cryopreserved and stored at the very low temperature of -196°C. Frozen cells are rapidly thawed before recovery and subsequent use as living cells. However, the survival rate of freeze-thawed cells is as low as 10 to 30% for other than tumor cells, such as Langerhans cells or liver cells, depending on the type of cells and the level of skill of the practitioner.

Furthermore, as blood cells and platelets cannot be cryopreserved, the shelf life thereof is as short as 12-72 hours. Additionally, in recent years, as the number of organ transplantations has increased, a method for preserving an organ to be transplanted has become an important issue of interest, and one which is closely linked to cell proliferation and cell disorder.

With recent advances in surgical technology, immunosuppression and the like, organ transplantation cases are increasing. During organ transplantation, it is ideal to immediately transplant an organ removed from a donor to a recipient, however, such transplantation operations are not always performed immediately. However, during transplantation operations it is extremely important to preserve precious organs, since such operations accompany an emergency. Current organ preservation methods include low-temperature methods, to suppress the metabolism of an organ, and perfusion of the organ to maintain metabolic function of the organ. A number of different varieties of preservation solutions used therefor have been developed and clinically applied.

In addition, when performing operations involving organs such as the heart, it may be necessary to transiently stop the blood supply, placing cells, tissues and organs in a state of ischemia, and when thereafter reperfusion is performed, free radicals are generated, disrupting the functions of said cells, tissues and organs. Conventionally, low-temperature treatment has been used to reduce such disorders, however, such treatment is complex, reduces the flexibility of the operation. Further, conventional treatment is often accompanied by disorders after reperfusion; thus it cannot be said that protection of cells, tissues and organs is fully achieved by present methodologies.

Recently, it has been found that polyphenols such as green-tea polyphenol and the like have physiological activity, including antioxidant, and such polyphenols have been widely studied for analyses and possible applications.

As described above, cells, tissues and organs accelerate lipid peroxidation of biological membranes in response to the presence of free radicals caused by ischemia, reperfusion and the like, causing biological membrane disorder, resulting in functional disorders of transplanted organs to be. In response to t this, we have been developing preservation liquids effective for inhibiting the cellular disorder caused by lipid peroxidation. Previously, we identified the inhibition of free radicals by polyphenols in cells, tissues and organs, and developed preservative agents for the cells, tissues or organs of an animal, comprising polyphenols as an active ingredient (Japanese Laid-Open Publication No. 2000-344602).

In Japanese Laid-Open Publication No. 2000-344602, a preservative agent comprising polyphenols is added to culture solution or organ preservative agent used for usual cell medium, in order to place cells removed from a living body into 'shut-down' status, to prolong the period of preservation by inhibiting growth thereof, or to maintain the functions of organs to be preserved for a long period of time.

When performing surgical procedures, including transplantation, there are a number of cases where targeted organs or the like must be maintained without a blood supply for a certain period of time.

In these cases, said organs are in an ischemic state because of the removal of the blood supply, which causes damage to biomembranes by the rapid production of free radicals, as described above. There is a time limit during surgical procedures, and significant damage arises during recovery from such surgery.

Specifically, for example, during cardiac surgery, cardioplegia is perfused in to the heart to cause artificial heart arrest; where heart dysfunction is often caused due to the ischemic state of cardiac muscle caused by insufficient oxygen. After induction of such an ischemic state, serious damage may occur to the cardiac muscle when reperfusion starts. This is called reperfusion damage, which may cause fatal damage to patients, and is deemed to be a serious problem.

In order to avoid or suppress such a state, the body temperature is lowered to reduce the rate of metabolism, and thus prolong the time available for surgery and reduce damage of organs and the like after surgery. It is still difficult to obtain sufficient periods of time for surgical procedures, and it cannot be said that the suppression of damage to organs is sufficient. Further, low temperature treatment requires complicated facilities, and is thus a drawback during operations during which precise treatment must be rapidly performed.

As described above, polyphenols have been found to be preservative at the cellular or tissue level. However, it cannot be expected that such "protective" functions will be maintained at the organ level, which is a high-order level. This is because, for example, it was well-known in the art that tissues removed from a dead body within 24 hours maintain their function. However, organs such as the heart, must be removed from the body, and thus removal of organs after brain death but before cardiac arrest plays an important role. Accordingly, a composition for "protecting" an organ, tissue or cell has not yet been found.

One object of the present invention is to suppress free radical production during the ischemic state of a cell, tissue or organ, and to maintain the function thereof (that is, the "protection" of organs, tissues and cells), and to prolong the period of time available for surgical procedures in ischemic states, improve recovery from post-surgery, and to prevent damage therefrom. Further, it is another object of the present invention to protect organs (particularly the heart) during preservation.

### SUMMARY OF INVENTION

The present invention is attained in part by our discovery that polyphenols have unexpectedly significant protective action in organs, tissues or cells.

Accordingly, the present invention provides the following:
1. A composition for protecting and preserving an organ, a tissue, or a cell, comprising a polyphenol.
2. The composition according to Item 1, wherein the polyphenol is present in an amount sufficient for preservation of the organ, tissue or cell, during ischemia or reperfusion.
3. The composition according to Item 2, wherein the ischemia and reperfusion arises in surgery or medical operation.
4. The composition according to Item 1, wherein the preservation is for operation, and the polyphenol is administered during the time from pre-operation to post-reperfusion.
5. The composition according to Item 4, wherein the administration is administered at a time from at least two weeks before an operation to the date of the operation.
6. The composition according to Item 4, wherein the administration is administered at least at the time of ischemia.
7. The composition according to Item 4, wherein the administration is performed at least on reperfusion.
8. The composition according to Item 4, wherein the administration is performed at least after reperfusion.
9. The composition according to Item 4, wherein the administration is performed orally or parenterally.
10. The composition according to Item 4, wherein the operation is surgical or internal.
11. The composition according to Item 10, wherein the operation is surgical.
12. The composition according to Item 11, wherein the surgical operation uses off-pump, PCI, catheter intervention or extracorporeal circulation.
13. The composition according to Item 11, wherein the surgical operation is an operation on the aorta, arteria coronaria or valve.
14. The composition according to Item 1, wherein the organ, tissue or cell is an organ.
15. The composition according to Item 1, wherein the organ comprises the skin, blood vessels, cornea, kidney, heart, liver, umbilical cord, intestine, nerve, lung, placenta, or pancreas.
16. The composition according to Item 1, wherein the organ is selected from the group consisting of the heart, brain, nerve and spinal cord.
17. The composition according to Item 1, wherein the organ, tissue or cell is mammalian.
18. The composition according to Item 1, wherein the organ, tissue or cell is human.
19. The composition according to Item 1, which is a protective agent for the organ, tissue or cell.
20. The composition according to Item 19, wherein the protection comprises protection during ischemia.
21. The composition according to Item 1, which is a preservation agent for the organ, tissue or cell.
22. The composition according to Item 1, wherein the polyphenol is a mixture or a single component.
23. The composition according to Item 1, wherein the polyphenol is a catechin, tannin, proanthocyanidine, or resveratrol.
24. The composition according to Item 1, wherein the polyphenol comprises at least catechins or tannins.
25. The composition according to Item 1, wherein the polyphenol comprises epigallocatechingallate.
26. The composition according to Item 1, wherein the polyphenol has a hydroxy value of about 2 to about 100.
27. The composition according to Item 1, wherein the polyphenol comprises an extract selected from the group consisting of tea extract, seaweed extract, aloe extract, wine extract, cactus extract and fruit extract.
28. The composition according to Item 1 wherein the polyphenol comprises seaweed extract.
29. The composition according to Item 1, wherein the polyphenol comprises tea extract.
30. A method for protecting an organ, tissue or cell in a subject, comprising:
   1) exposing the organ, tissue or cell to a polyphenol.
31. The method according to Item 30, wherein the polyphenol is administered to the subject in an amount effective for protecting the organ, tissue or cell, during ischemia or reperfusion.
32. The method according to Item 30, wherein the protection is performed at operation.
33. The method according to Item 30, wherein the operation is surgical or internal.
34. The method according to Item 32, wherein the polyphenol is administered to the subject at any period of time from pre-operation to reperfusion.
35. The method according to Item 34, wherein the administration is performed at any time point from at least two weeks before operation to the date of operation.
36. The method according to Item 34, wherein the administration is performed at least at ischemia.
37. The method according to Item 34, wherein the administration is performed at least at reperfusion.
38. The method according to Item 34, wherein the administration is performed at least after reperfusion.
39. The method according to Item 34, wherein the administration is performed orally or parenterally.
40. The method according to Item 32, wherein the operation is surgical.
41. The method according to Item 32, wherein the surgical operation uses off-pump, PCI, catheter intervention or extracorporeal circulation.
42. The method according to Item 40, wherein the surgical operation is an operation on the aorta, arteria coronaria or valve.
43. The method according to Item 30, wherein the organ, tissue or cell is an organ.
44. The method according to Item 30, wherein the organ comprises the skin, blood vessels, cornea, kidney, heart, liver, umbilical cord, intestine, nerve, lung, placenta, or pancreas.
45. The method according to Item 30, wherein the organ is selected from the group consisting of the heart, brain, nerve and spinal cord.
46. The method according to Item 30, wherein the organ, tissue or cell is mammalian.
47. The method according to Item 30, wherein the organ, tissue or cell is human.
48. The method according to Item 30, wherein the protection comprises protection during ischemia.
49. The method according to Item 30, wherein the protection comprises protection at reperfusion.
50. The method according to Item 30, wherein the polyphenol is a mixture or a single component.
51. The method according to Item 30, wherein the polyphenol is a catechin, tannin, proanthocyanidine,or resveratrol.
52. The method according to Item 30, wherein the polyphenol comprises at least catechins or tannins.
53. The method according to Item 30, wherein the polyphenol comprises epigallocatechingallate.
54. The method according to Item 30, wherein the polyphenol has a hydroxy value of about 2 to about 100.
55. The method according to Item 30, wherein the polyphenol comprises an extract selected from the group consisting of tea extract, seaweed extract, aloe extract, wine extract, cactus extract and fruit extract.
56. The method according to Item 30, wherein the polyphenol comprises seaweed extract.
57. The method according to Item 30, wherein the polyphenol comprises tea extract.
58. A method for preserving an organ, tissue or cell, comprising the step of:
   1) retaining the organ, tissue or cell in a fluid comprising a polyphenol.
59. The method according to Item 58, wherein the organ is the heart.
60. Use of polyphenol in manufacturing a medicament for protecting or preserving an organ, tissue or cell, wherein the medicament comprises the polyphenol.

Accordingly, the present composition may be a metabolic function suppression agent comprising a polyphenol as an active ingredient, which is administered directly or indirectly to the living body to attain protection (i.e. restoring metabolism function to a normal state) of a cell, tissue or organ. The present composition may also be metabolic function enhancing agent, comprising a polyphenol as an active ingredient, which is administered to the living body in advance of a surgical procedure during which the blood flow to an organ is interrupted in order to bring the target organs into a state of suppressed metabolic function.

Further, the present composition is characterized in it's use for suppressing ischemic disorder of an organ arising during a surgical operation which interrupts the blood flow to an organ, by administering in advance to the living body a metabolic function suppression agent, comprising a polyphenol as an active ingredient, to restore the metabolic function of the target organ to a normal state. More specifically, in one embodiment, it is a method to suppress ischemic disorder, characterized in that the above-mentioned surgical procedure is a heart operation, and the targeted organ is a heart, wherein the administration method is oral and i.v. administration. Further, the method to suppress ischemic disorder further provides administration conditions during which administration is performed 0.01-0.1g/kg/day to achieve protection of cardiac muscle of the heart.

Hereinafter the preferable embodiments of the present invention are described. However, it should be appreciated that those skilled in the art can readily and appropriately carry out such embodiments of the invention from the description of the present invention and the well-known technologies and common general knowledge in the art, and readily understand the effects and advantages of the present invention therefrom.

### BRIEF DESCRIPTION OF THE DRAWINGS

**Figure 1** is an enlarged photograph of myocardium pathological specimens showing heart cross-sections from the control rat group and the polyphenol-administered rat group.
**Figure 2** is a graph showing the end-systolic pressure-volume relationship of a ventriculus sinister.
**Figure 3** shows the results of determining immunopathology using 8-OHdG.
**Figure 4** shows an enlarged photograph of a pathological specimen showing vacuolar degeneration of the nucleus of a myocardial cell (control).
**Figure 5** shows an enlarged photograph of a pathological specimen showing vacuolar degeneration of the nucleus of a myocardial cell (polyphenol administered).
**Figure 6** shows a photograph showing polarized distribution of intracellular polyphenol using an FITC labeled polyphenol.
**Figure 7** shows the effect of a polyphenol on the weight of a heart after reperfusion.

### DESCRIPTION OF PREFERRED EMBODIMENTS

Hereinafter the present invention is described. It should be understood throughout the present specification that articles for singular forms (e.g., "a", "an", "the", etc. in English) and articles, adjectives, etc. include plural referents unless the context clearly dictates otherwise. It should also be understood that the terms as used herein have definitions typically used in the art unless otherwise mentioned. Accordingly, unless otherwise defined, all the technical terms and scientific terminology as used herein will have the same meaning as those usually understood by those skilled in the art. If there is contradiction, the present specification (including the definition) takes precedence.

Hereinafter, the definitions of terms particularly used herein are listed.

As used herein the term "polyphenol" refers to a phenol having at least two hydroxy groups in the same molecule, also known as a "multivalent phenol". Depending on the number of the hydroxy groups, they are named dihydric phenol, trihydric phenol, and the like. For example, C₆H₄(OH)₂ such as cathechol, resorcin, hydroquinone and the like are dihydric phenols, and C₆H₃(OH)₃ such as fluoroglucin and the like are trihydric phenols. Polyphenols are classified into flavonoids, hydrolyzed tannins, and other polyphenols, according to a classification method. Hydrolyzed tannins and proanthocyanidine (reduced tannins) are collectively called tannins (or tannic acids). Preferable polyphenols, as preferably used herein, are those previously approved as a food or a pharmaceutical (including quasi drugs), including, but not limited to, for example, tannic acids, albumin tannate and the like, which are listed in the Japanese Pharmacopoeia.

As used herein the term "tannins" collectively refer to chemicals having polyoxyphenyl as a basic structure, and that produce phenols when alkaline hydrolyzed. They are classified into pyrogallol tannins, that produce pyrogallol upon potash fusion, and cathechol tannins, that produce cathechol upon potash fusion. Further included are hydrolyzing tannins that produce gallic acid and ellagic acid upon hydrolyzation by heating with diluted acid, and reduced tannins that produce probaphenone upon polymerization, which is water soluble. Hydrolyzing tannins often have structure in which polyoxydiphenic acid is bound to a sugar via depside linkage. Reduced tannins are believed to be produced by the polymerization of a number of monomers such as catechins, leukoanthocyanins and the like. As used herein, any type of tannins may be effective. In one embodiment, the tannins may be tannic acid or albumin tannate. Tannic acid is a crude product of gallotannins, and is used as a pharmaceutical. Albumin tannate is a mixture of tannins and albumin, and is also used as a pharmaceutical.

As used herein the term "flavonoid" collectively refers to a group of pigments having C6-C3-C6 carbon backbones. It is a collective reference to derivatives of flavanes. A number of polyphenols are produced in plants, and are often biosynthesized from malonyl-CoA and cinnamic acid in plant bodies. Flavonoids used herein include calchons, flavanones, flavones, flavonols, flavanonols, flavanols (catechins), isoflavones, anthocyanins, benzalcoumaranones, anthocyanidines, protocyanidines, and the like. Flavonoids have antioxidant properties, and generally, as the number of the phenolic hydroxic group reduces, the effects thereof become weaker. Flavonoids are noted for their antitumor activity.

Such flavonoids typically have the following structure:

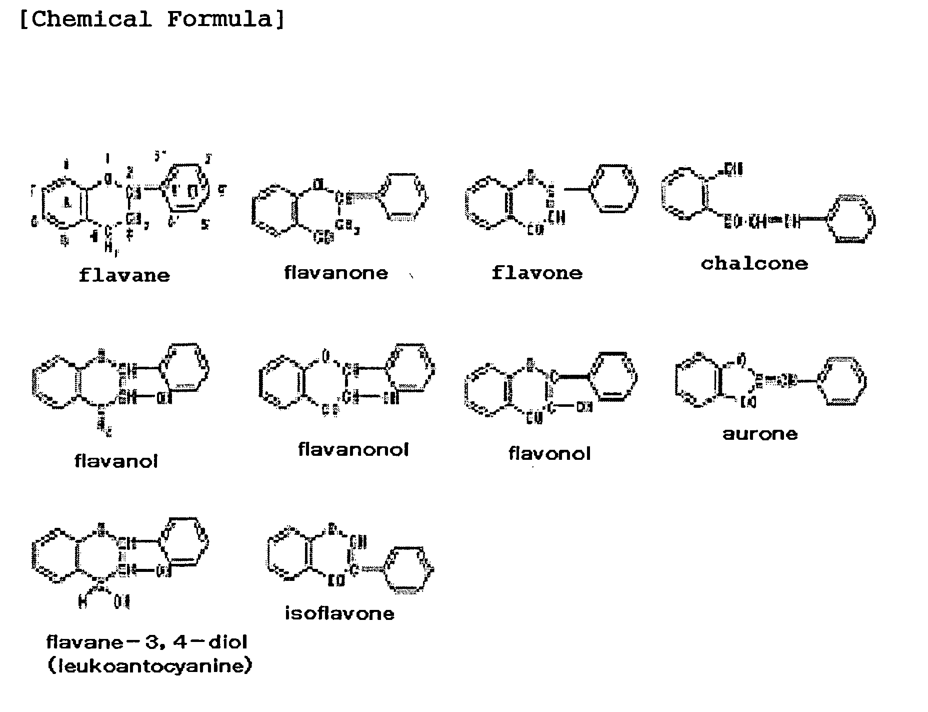

As used herein the term "chalcone" refers to 1,3-diphenyl-2-propylene-1-one (1,3-diphenylprop-2-ene-1-one), and is also known as benzalacetophenone. Chalcones encompass hydroxy derivatives of chalcone. Chancones include, but are not limited to, in addition to chalcone, buteincoreopsin, isobu, chalconocaltamidine, isocarthamine, carthamine, bedicine, bedicinine and the like.

As used herein the term "flavanone" refers to 2,3-dihydroflavone, and flavanones collectively refer to derivatives thereof (for example, hydroxy derivatives and methoxy derivatives (in particular, 3,5,7,3',4, 5 positions are substituted). Flavanones are mainly present in plant kingdom (in particular, oranges) as a glycoside. Flavanones include, but are not limited to, in addition to flavanone, pinocembrine, naringenin, saliburbine, burnin, naringin, sakyranetine, sakuranine, hesperitin, hesperidine, eriodictyol, matisynol and the like.

As used herein the term "isoflavone" refers to 3-phenyl chromone, and isoflavones further encompass derivatives and glycosides thereof. Isoflavones include in addition to isoflavone, but are not limited to, daidzein, daidzin, genistein, genistin, and the like.

As used herein, the term "flavone" refers to 2-phenyl chromone (C₁₅H₁₀O₂), and flavones collectively refer to derivatives thereof (for example, hydroxy derivatives, methoxy derivatives and the like). Flavones include in addition to flavone, but are not limited to, chrysin, toringin, apigenin, cosmocyin, abiyne, luteolin, galtheorin, glucortheoline, and the like.

As used herein the term "flavonol", refers to 3-hydroxyflavone (C₁₅H₁₀O₃), and flavonols collectively refer to derivatives thereof (for example, glycosides, hydroxy derivatives, and methoxy derivatives). Flavonols include kenpherol, tripholine, astragallin, robinin, quercetin, quercitrine, isoquercitrine, rutin, myricetin, myricitrin and the like.

As used herein the term "flavanonol" refers to a flavanone in which a hydroxy group is bound to position 3 of the C-ring, and flavanonols collectively refer to derivatives thereof. Flavanonols include in addition to flavanonol, but are not limited to, pinobanksin, aromadendrine, engelitin, fustin, taxiforin, astilbin, ampeloptin, and the like.

As used herein the term "flavanol (catechin)" refers to a flavane in which a hydroxy group is bound to position 3 of the C-ring, and catechins collectively refer to derivatives thereof. Catechins include in addition to catechin, but are not limited to, gallocatechin, epicatechin, epigallocatechin, epicatechingallate, peigallocatechingallate, and theaflagines, in which two molecules are dimerized, such as theaflavine, theaflavine-3-O-gallate, theaflavine-3'-gallate, theaflavine-3,3'-dl-O-gallate, and the like). Catechins are often contained in teas.

As used herein the term "anthocyanidine" refers to an aglycon in which 4-6 hydroxy groups are bound to a 2-phenyl benzopyrylium structure. A glycoside thereof refers to anthocyanin, and anthocyan collectively refers to both.

Benzalcoumaranone (aurone) refers to C₁₅H₁₀O₂. Benzalcoumaranones collectively refer to derivatives thereof. Benxalcoumaranones include, but are not limited to, in addition to bencalcoumaranone, sulphurein, sulphurein, paracitrineleptosidine, leptocine, aureusidin , aureusin, cernuoside, and the like.

The present invention may target any organs, and the tissues or cells to be targeted by the present invention may be derived from any organ of an organism. As used herein, "organ" refers to a structure which is a specific portion of an individual organism, where a certain function of the individual organism is locally performed and which is morphologically independent. Generally, in multicellular organisms (e.g., animals and plants), organs are made up of several tissues in a specific spatial arrangement and tissue is made up of a number of cells. Examples of organs or parts include organs or parts related to the circulatory system. In one embodiment, examples of organs targeted by the present invention include but are not limited to the skin, blood vessels, cornea, kidney, heart, liver, umbilical cord, intestine, nerve, lung, placenta, pancreas, brain, extremities, retina and the like. Preferably, the organ targeted by the present invention is the heart. In another preferred embodiment, the organs targeted by the present invention are the liver, kidney and the like..

As used herein, the term "tissue" refers to a group of cells having the substantially same function and/or form in a multicellular organism. Generally, the term "tissue" has the same origin but may have different origin, as long as the tissue has the same function and/or form, and such may also be called as "tissue" herein. Accordingly, when regenerating tissue using a stem cell in the present invention, a group of cells having two or more different origins may constitute one tissue. Usually, tissues constitute parts of organs. Animal tissues are classified into epidermal tissues, connective tissues, muscular tissues, nervous tissues, and the like, based on morphological, functional or developmental grounds. In plants, tissues are classified into meristem and permanent tissues. Tissues are also classified into single tissue and complex tissues, depending on the types of constituting cells, and the like.

As used herein, the term "cell" is defined as having the widest meaning used in the art, referring to a structural unit of multicellular organisms, which has an enveloping membrane structure for separating the cell from the outside, has self-regeneration capability, and which is a living body having genetic information and an expression mechanism. The cells which can be targeted herein include cells in an *in vivo* state. Cells include for example, epidermal cells, pancreas parenchymal cells, pancreatic duct cells, liver cells, blood cells, myocardium cells, skeletal muscle cells, osteoblasts, skeletal muscle osteoblasts, nerve cells, blood vessel endothelial cells, pigment cells, smooth muscle cells, adipocytes, bone cells, chondrocytes, and the like. Cells can be classified based upon the stem cells from which they derive, including ectoblasts, mesoblasts, and endoblasts. Cells from ectoblasts are present mainly in the brain, and include nerve stem cells. Cells from mesoblasts are present mainly in the bone marrow, and include blood vessel stem cells, hemeatopoietic stem cells, mesenchymal stem cells and the like. Cells from endoblasts are mainly present in visceral organs, and include liver stem cells and pancreatic stem cells. Somatic cells, as targeted herein, may be any cells from any blastodermic layer.

As used herein, the term "protection" of an organ, tissue or cell, stopping the elimination of function of the organ, tissue or cell is stopped, without harming the organism *in vivo,* preferably, the function is maintained, more preferably improved. For example, protection of heart refers to an act by which the heart is protected from ischemic disorders and the like, and cardiac dysfunction is prevented. In particular, a variety of factors present in the living organism may lead to disorder, such as cell necrosis after ischemia, and free radical production during reperfusion thereafter, and thus protecting organs, tissues or cells from such factors is encompassed in the concept of protection.

As used herein, the term "preservation" of organs, tissues or cells, refers to maintenance or improvement of the state of an organ, tissue or cell *in vitro.* Accordingly, protection and preservation are different in their concept. In particular, with respect to substances which are recognized to have preservative effects, it is not possible to predict that such substances act in the same manner *in vivo,* generally speaking.

As such, in some aspects, preservation and protection have similar properties, however, in "preservation", once preservation processing has been performed, such a sample may be left and may be allowed to stand for a long period of time. On the other hand, if protection is performed for a long period of time, it may adversely affect the living organism per se, and therefore long term processing is not performed.

As used herein the terms "ischemia" and "ischemic state" are interchangeable used to refer to a partial or total lack of local blood supply to an organ or tissue. It causes local damage to, or death of, a tissue as a result of disruption to the blood supply. When performing cardiac operations, it is necessary to partially or entirely disrupt the blood supply for a certain period of time, and in that case, the heart becomes ischemic for at least a certain period of time, and thereafter reperfusion is performed, causing further disorders. Conventionally it has been to protect the heart in an ischemic state.

As used herein, the term "reperfusion" refers to re-establishing the blood supply after opening of a coronal artery occlusion. Reperfusion is accompanied by reperfusion disorders. Reperfusion disorders include myocardium disorders following opening of an coronal artery occlusion, and are often accompanied by arrhythmia. This is due to free radicals from oxygen.

As used herein, the term "ischemic disorder" encompasses disorders caused during ischemia and reperfusion, and include but are not limited to disorders and diseases due to ischemic necrosis, paralysis, heart failure, myocardium deliquium, myeloparalysis, retinopathy, optic nerve disorders, ischemic cardiomyopathy, organ ischemic disorders such as brain ischemic disorders, renal ischemic disorders, hepatic ischemic disorders and the like.

A "disease" targeted by the present invention may be any disease in which tissue is injured. Examples of such a disease may be any disease of any organ, including, but not limited to, heart diseases such as heart failure, myocardial infarction, cardiomyopathy, and the like. The protection method of the present invention is applied to the protection of an organ other than the heart.

The term "heart failure" refers to the inability of the heart to circulate blood in a required quantity and quality to organs in the entire body due to an impairment of the heart itself, such as failure of cardiac functions, failure of circulatory functions, a reduction in contractile power, or the like. Heart failure is a terminal symptom of heart diseases, such as myocardial infarction, cardiomyopathy, and the like. Severe heart failure means that the state of the heart is severe and is also referred to as terminal heart failure.

The term "myocardial infarction" refers to a disease in which ischemic necrosis occurs in a perfusion area, associated with highly developed constriction or occlusion caused by various lesions of the coronary artery. The severity of myocardial infarction is divided into classes in various manners. Classification may be based on, for example, progress over time; morphology (e.g., the range, site, necrosis size, or the like within the myocardium); the necrosis form of a myocardium; the reconstruction of a ventricle after infarction; the dynamics of blood circulation (associated with therapy, prognosis, etc.); clinical severity; and the like. Myocardial infarction having a high level of severity is particularly called severe myocardial infarction.

The term "cardiomyopathy" is a generic term for diseases caused by organic and functional abnormality in a myocardium, which are divided into secondary cardiomyopathy following a basic disease (e.g., hypertension, dysbolism, ischemia, etc.), and spontaneous cardiomyopathy which develops without an apparent basic disease. As a pathological change, myocardial hypertrophy, formation of fibrous tissue, degeneration, or the like is observed.

As used herein, the term "prophylaxis" or "prevention" in relation to a certain disease or disorder, refers to a treatment which prevents such a condition from happening in the first instance, or causes the condition to occur at a reduced level or to be delayed. The protection method of the present invention is thus used for prevention or is combined with another prevention method.

As used herein, the term "therapy" in relation to a certain disease or disorder, means that when such a condition occurs, such a disease or disorder is prevented from deteriorating, preferably is retained as it is, more preferably is diminished, and even more preferably is extinguished. The protection method of the present invention may be combined in therapy.

As used herein the term "off-pump", when used in surgical operation, refers to treatment without an artificial heart lung machine. An off-pump operation is employed to treat the coronal artery. An off-pump operation causes transient ischemia locally when a blood vessel is ligated for 10-30 minutes, and the dysfunction caused thereby often leads to problems.

As used herein the terms "extracorporeal circulation", "pump" or "artificial heart lung" are interchangeably used to refer to treatment using an artificial heart lung machine. When using an artificial heart lung machine, problems occur during ischemic and reperfusion periods, and thus ischemic disorders have become a problem for any operation.

As used herein the term "operation" may be surgical or internal. Surgical operations include, but are not limited to, for example, AC by-pass, valvoplasty, cardiac valve replacement, aorta operation, living donor liver transplantation, living-donor kidney transplantation, brain-death liver transplantation, heart transplantation and the like. Preferably, operations performed in the present invention, are performed on the aorta, coronal artery or squama. Internal operations include, for example, PCI (for example, PTCA) using a balloon catheter, catheter intervention, PTA against different types of blood vessels, and the like. Balloon catheter techniques often substantially falls within the category of off-pump operation.

Cells, tissues or organs targeted by the present invention include any organism as long as it has organs (e.g., multicellular organisms such as animals (e.g., vertebrates, invertebrate), plants (e.g., monocot, dicot) and the like). Preferably, the animal is a vertebrate (e.g., Myxiniformes, Petronyzoniformes, Chondrichthyes, Osteichthyes, amphibian, reptilian, avian, mammalian, etc.), more preferably mammalian (e.g., monotremata, marsupialia, edentate, dermoptera, chiroptera, carnivore, insectivore, proboscidea, perissodactyla, artiodactyla, tubulidentata, pholidota, sirenia, cetacean, primates, rodentia, lagomorpha, etc.). More preferably, primates (e.g., chimpanzee, Japanese monkey, human, etc.) are targeted. Most preferably, a human is targeted.

When the present invention is used as a pharmaceutical agent, it may further comprise a pharmaceutically acceptable carrier or the like. A pharmaceutically acceptable carrier contained in a medicament of the present invention includes any material known in the art. Examples of such a pharmaceutically acceptable carrier include, but are not limited to, antioxidants, preservatives, colorants, flavoring agents, diluents, emulsifiers, suspending agents, solvents, fillers, bulking agents, buffers, delivery vehicles, excipients, pharmaceutical adjuvants, and the like. Typically, the pharmaceutical composition used in the present invention is administered in a form of composition comprising one or more types of polyphenols (for example, a mixture), with at least one physiologically acceptable carrier, excipient, or diluent. For example, an appropriate vehicle may be water for injection, a physiological solution, or an artificial cerebro-spinal cord solution, and may be supplemented with other substances which are generally used for parenteral delivery of a composition.

Examples of appropriate carriers include neutral buffered saline or saline mixed with serum albumin. Preferably, the product is formulated as a lyophilizate using appropriate excipients (e.g., sucrose). Other standard carriers, diluents, and excipients may be included as desired. Other exemplary compositions comprise Tris buffer of about pH 7.0-8.5, or acetate buffer of about pH 4.0-5.5, which may further include sorbitol or a suitable substitute therefor.

The pharmaceutical composition of the present invention may be administered parenterally. Alternatively, the present composition may be administered i.v. or percutaneously. When systemically administered, the pharmaceutical composition used in the present invention may be in a pharmaceutically acceptable aqueous form, which does not include pyrogens. Preparation of such pharmaceutically acceptable compositions is within the capabilities of one skilled in the art, providing that substantive attention is paid to the survival of a cell, tissue or organ, with regard to the composition's pH, isotonicity, stability, and the like.

The therapeutic formulation of the present invention may be prepared for storage by mixing a selected composition, having the desired degree of purity, with optional physiologically acceptable carriers, excipients, or stabilizers (as described in the Japanese Pharmacopeia; Remington's Pharmaceutical Sciences, 18th Edition, A. R. Gennaro, ed., Mack Publishing Company, 1990; and the like), in the form of a lyophilized cake or an aqueous solution.

Acceptable carriers, excipients or stabilizers used herein are preferably nontoxic to recipients and are preferably inert at the dosages and concentrations employed, and preferably include phosphate, citrate, or other organic acids; antioxidants (-e.g., ascorbic acid); low molecular weight polypeptides; proteins (e.g., serum albumin, gelatin, or immunoglobulins); hydrophilic polymers (e.g., polyvinylpyrrolidone); amino acids (e.g., glycine, glutamine, asparagine, arginine or lysine); monosaccharides, disaccharides, and other carbohydrates (glucose, mannose, or dextrins); chelating agents (e.g., EDTA); sugar alcohols (e.g., mannitol or sorbitol); salt-forming counterions (e.g., sodium); and/or nonionic surfactants (e.g., Tween, pluronics or polyethylene glycol (PEG)).

### DESCRIPTION OF BEST MODE OF THE PRESENT INVENTION

In one embodiment, the present invention provides a composition for the protection and preservation of an organ, tissue or cell, comprising a polyphenol. The polyphenol is present at a pharmaceutically or medically (or agriculturally or veterinary) effective concentration.

We became interested in polyphenols several years ago, with respect to their inhibitory action on cancer cell growth, and studied a number of their properties. The present invention has unique properties which are not recognized in conventional antioxidants (for example, superoxide dismutase (SOD) as an oxygen system, Vitamin E, C, glutathione, carotinoids, flavonoids, sugars, iron chelates, uric acid, albumines and the like), such as amphipathicity (i.e. high solubility in water and organic solvents), good absorbance against proteins, extremely low cytotoxicity, and ten fold or greater antioxidant activity than that of SOD. Further, we also found that the present invention can flexibly control the growth of an animal cell, which has not been previously known in the art.

Since the 1908's polyphenols have been reported to have a variety of good physiological actions, such as anti-tumor action, antioxidant action, antibacterial and antiviral actions, by a number of different researchers. Polyphenols have also already been reported to have adverse effects on cellular proliferation, however, most of these effects are directed to suppression of tumor cell proliferation.

Two Nature papers have recently been noted: J. Jankun, S. H. Selman, and R. Swiercz, "Why drinking green tea could prevent cancer", Nature, 387, 5 June,561,1997, and Y, Cao and R. Cao, "Angiogenesis inhibited by drinking tea, Nature, 398, 1 April, 381, 1999.

Green tea polyphenols have a variety of actions such as antioxidant, deodorant, antibacterial actions and the like, and in addition have cariostatic or other physiological activities (Inshokuryohinyokinosei sozai yuko riyo gijutsu to riizu No. 10, ryokucha porifenooru, Uemura mitsuo, Kashisogo gijutsu center, San-yu-sha). Thus, recently, green tea polyphenols have been preferably taken as a functional food. However, their preservation activity in animal cells or organs have not been suggested to date.

As such, it appears that most research has conventionally been directed to the anti-tumor action of polyphenols as antioxidants.

In such circumstances, we have recently found that polyphenols have preservation effects *in vitro*, on cells or tissues, and have filed a patent application. In this application, isolated cells or tissues and organs resected from living body, and soaked in a preservation solution for blocking and preservation thereof, or alternatively a preservation agent is administered as a post-operative treatment , to attempt to prevent a variety of disorders occurring within the organs and to facilitate recovery therefrom.

In this case, if the concentration of polyphenols in the preservation solution or blood is maintained within an effective, it was expected that the production of free-radicals in cells would be suppressed, thus providing the mechanism of action for the current invention.

Accordingly, such actions and effects attained by maintaining the blood-polyphenol concentration within a certain range, are also attained by soaking organs, tissues or cells in a preservation solution or administering the same. However, of course, in the ischemic state, no such effects were expected to occur, and it was rather believed that the actions and effects of polyphenols were transient, regardless of the mode of administration.

We have discovered that when administering polyphenols to a living body, the subsequent course of events with respect to the actions and effects of polyphenols on cells, tissues or organs, is not always the same as compared with soaking said cells, tissues or organs in a preservation solution with polyphenols added thereto, but rather, when administered to the body, the protective effects on cells, tissues, and organs form the basis of the present invention.

That is, if polyphenols having these activities are continuously administered over a certain period of time to a target animal, thereafter, if the blood supply to these cells, tissues and organs is disrupted by removing and/or resecting them from the body, these cells, tissues or organs still retain metabolic function. Thereafter, even if reperfusion is performed, metabolic function is recovered (or maintained), and no damage is caused by free radicals. Accordingly, in particular, the present invention attains a significant effect, which is not attainable from the conventional methods, in terms of organ protection during ischemia.

Accordingly, in such states, it is possible to disrupt the blood flow to living tissues or organs or the like for a long period of time for surgical procedures. Such effects are not attainable by conventional technologies, and thus the utility thereof should be acknowledged.

Products having 60 % or more pure polyphenols are usually available, and products having 60 % or more purity may be used as a preservative agent for cell or organ of the present invention, purified products having 85 % or more purity are more suitable. Of course, products having even greater purity are more preferable.

Accordingly, polyphenols contained in the composition of the present invention, encompass any polyphenols regardless of the purity thereof. In particular, preferable polyphenols are catechins or tannins, amongst them, the catechin known as 3,3,4,5,7-flavopentol, cathecolamine having 3,4-dihydroxy phenyl backbone, noradrenaline, adrenaline, dopamins and the like, and catechins having epigallocatechingallate as main components are particularly preferable.

Examples of other preferable polyphenols include tannic acid. Generally, for example, pharmaceutical grade tannic acid configures eight gallate groups around a glucose on the same plane, and binds two gallate groups in the perpendicular direction. However, the center of the compound is not always a glucose but may also be a cellulose compound, an didebuside gallate obtained by hydrolysis of a tannic acid may be used herein.

Accordingly, polyphenols used in the present invention may be a mixture or a pure product. In preferable embodiments, polyphenols are selected from the group consisting of catechins, tannins, proanthocyanidine, and risberatrol.

In one preferable embodiment, polyphenols comprise at least catechins and tannic acid. In more preferable embodiment, polyphenols comprise epigallocatechin gallate. In another preferable embodiment, polyphenols comprise polyfluoroglucinol complex. Tea extract, seaweed extract, wine extract, cactus extract and fruit extract are preferable.

The hydroxy value of polyphenols used in the present invention may be any number as long as the number suits the definition of polyphenols. Usually, the hydroxy value may be 2 to about 100, preferably at least 3, more preferably at least 4.

Polyphenols as used herein may be extracted from a variety of foods. Such foods include, but are not limited to tea, wine, chocolate, cactus, seaweed, vegetables, onions (the dark yellow membrane of most outer part), aloe extract, leaves of parsley, white vegetables and the like), fruits such as oranges (Satsuma, *daidai* orange, *ponkan* orange skin, *natsumikan* orange skin, grapefruits, lemon, and the like), apples and the like, cereals (kaoliang, soybean, buckwheat, wheat and the like), and flowers such as dahlia and the like. Preferably, polyphenols include an extract selected from the group consisting of tea extract, seaweed extract, fruit extract, cactus extract, and wine extract.

In one preferable embodiment, polyphenols comprise seaweed extract. In another embodiment, polyphenols comprise tea extract.

In a preferable embodiment, the present invention is administered at least during ischemia and/or reperfusion, in an amount effective for the protection of an organ, tissue or cell. In order to attain such conditions, polyphenols may be administered pre-operatively, or at the time of ischemia or reperfusion. Preferably, administration is performed at least at any of the following time points, such as at least two weeks before operation (alternatively, at least one week before operation, at least three days before operation, at least two days before operation, at least one day before operation and the like), to the date of operation. More preferably, administration is performed periodically from about two weeks before operation to the date of operation (for example, daily, 0.1-100 mg/kg weight, such as 40mg/kg weight, per day may be performed). In another embodiment, the administration is performed at least at the time of ischemia. In another embodiment, the administration is performed at least at the time of reperfusion. In still other embodiment, the administration is performed after the reperfusion. The time span of administration is not limited to such preferable embodiments, and the frequency of performing the method of treatment of the present invention on a subject or a patient may be readily determined by those skilled in the art, in consideration of the purpose of use, targeted diseases (type, severity and the like), age, weight, sex, disease history of the patient, and the course of treatment. Frequencies include, for example, three to four times per day (for example, continuing for one to two weeks) to once per two weeks. Administration frequencies may be altered upon monitoring the course of treatment. For example, three times a day, 2-10 weeks, continuous administration, every time before meals, every time between meals, and the like are included but are not limited thereto. The administration of the composition of the present invention may be performed using well known methods in the art, and include oral or parenteral administration. Such parenteral administration methods may include, for example, iv administration, intramuscular administration, subcutaneous administration, intra-cutaneous administration, mucous administration, intrarectal administration, intravaginal administration, local administration to the diseased portion, local administration, and the like. Formulation for such administration may be provided in thee form of any formulation. Such formulation forms include, but are not limited to, for example, liquids, injection agents, sustained release agents, and the like. The amount of polyphenols used in the method of treatment of the present invention may be readily determined by those skilled in the art in consideration of the purpose of use, targeted disorders, operation, diseases (type, severity), age, weight, sex, disease history of a patient, forms and types of cells, and the like.

In certain embodiments, the above-mentioned ischemia or reperfusion may occur during surgical or internal operations. Accordingly, the invention attains significant effects for ischemic states which occur during surgical or internal operations.

In one embodiment of the present invention, the organ, tissue or cell to be protected may be a subject organ for a surgical operation. When such protection is particularly intended, polyphenols may be administered at any time point from pre-operation to after reperfusion, more preferably, within the above-mentioned administration frequency, time-span, timing and the like.

In one embodiment of the present invention, the operation may particularly be a surgical operation. Accordingly, organs are targeted by the present invention for protection. Surgical operations may be off-pump, PCI, catheter intervention or those using extracorporeal circulation, and in the case of surgical operations on the heart, for example, it may include, but is not limited to, for example, the aorta, coronal artery or squuam and the like. Accordingly, the present invention may target organs such as, for example, blood vessels, cornea, kidney, heart, liver, umbilical cord, intestine, nerve, lung, placenta or pancreas. Preferably, such organs include, but are not limited to, the brain, nerve, heart, kidney and the like.

The polyphenol containing composition of the present invention may be used for the purposes of protection or preservation in any organism as long as a living organism is treated. Preferably such organisms are vertebrates or invertebrates. Preferably, the organism is a vertebrate (e.g., Myxiniformes, Petronyzoniformes, Chondrichthyes, Osteichthyes, amphibian, reptilian, avian, mammalian, etc.), more preferably mammalian (e.g., monotremata, marsupialia, edentate, dermoptera, chiroptera, carnivore, insectivore, proboscidea, perissodactyla, artiodactyla, tubulidentata, pholidota, sirenia, cetacean, primates, rodentia, lagomorpha, etc.). Illustrative examples of a subject include, but are not limited to, animals, such as cattle, pigs, horses, chickens, cats, dogs, and the like. More preferably, primates (e.g., chimpanzee, Japanese monkey, human, etc.) are used. Most preferably, a human is used.

In preferable embodiments, the composition of the present invention is for the protection of an organ, tissue or cell. The protection includes, but is not limited to, protection during ischemic states. In another embodiment, the composition of the present invention is aimed at preservation of an organ, tissue or cell.

In another aspect, the present invention provides a method for protecting an organ, tissue or cell in a subject, comprising: 1) exposing the organ, tissue or cell to a polyphenol. In the subject method, polyphenol may preferably be administered to the subject at an amount effective for protecting the organ, tissue or cell, during at least one period of time selected from the group consisting of ischemia and reperfusion. The administration mechanism of the polyphenols in the protection method of the present invention, is any format, as mentioned above, as long as an amount is administered such that an effective concentration is present during the ischemic state, the reperfusion period, or when reperfusion disorders arise. Preferably, the administration is performed at any time point from pre-operation to post-reperfusion. Preferably, administration is performed at any time point from at least two weeks pre-operation to the date of operation, more preferably, is performed at least either during the ischemic state or during reperfusion. Alternatively, administration may be performed after reperfusion. Administration after reperfusion may attain a reduction or cure of reperfusion disorders. Administration prior to reperfusion attains preventative effects , and may further have an action to reduce disorders when such disorders occur.

In a protective method of the present invention, administration may be oral or parenteral. It is preferable to perform administration orally before an operation, with regard at least to patient convenience. During an operation, administration is preferably performed parenterally, as patients are usually anesthetized, thus precluding oral administration.

As used herein, the term "subject" refers to an organism to which the treatment of the present invention is applied and is also referred to as "patient". A patient or subject may be any organism as long as the present invention is applied, and is preferably a human.

In certain embodiments, the protection provided by the present method is used during operations. Amongst such operations which cause ischemic states, the method of the present invention has an effect. Operations may be surgical or internal operations. The method of the present invention is particularly suitable for surgical operations. Such surgical operations may be off-pump, PCI, catheter intervention, or extracorporeal circulation, and such surgical operations may be operations on the aorta, coronal artery or squama.

In preferable embodiments, the method of protection of the present invention may be a method for protecting an organ. Organs include, but are not limited to, the skin, blood vessels, cornea, kidney, heart, liver, umbilical cord, intestine, nerve, lung, placenta, pancreas and the like. Preferably, the organs are selected from the group consisting of the heart, brain, nerve and spinal cord.

The organs, tissues or cells to be targeted by the protection method of the present invention may be any organism, and in particular preferably those of a mammal, and more preferably those of a human.

The protection method of the present invention may include at least either protection during ischemia or during reperfusion.

The polyphenols used in the protective method of the present invention may be as described hereinabove, and a variety of polyphenols may be used.

In another aspect, the present invention provides a method for preserving an organ, tissue or cell. The present method comprises: 1) retaining the organ, tissue or cell in a fluid comprising a polyphenol. In the present invention, in particular, it was found that polyphenols have not only protective effects on the heart, but also preservation effects. This has not yet reported in the prior art, and thus can be said to be a significant effect.

In another aspect, the present invention provides a use for polyphenol in manufacturing a medicament for protecting or preserving an organ, tissue or cell, wherein the medicament comprises the polyphenol.

In the present invention, when the medicament of the present invention is provided as a package, instructions may be attached. Such instructions are prepared according to a format defined by the domestic authority for which the present invention is provided (such as the Ministry of Labour, Health, and Welfare in Japan, Food and Drug Agency (FDA) in the United States), and expressly describes that the product is approved by the domestic authority. The composition of the present invention may usually be worked under the supervision of a medical doctor, however, depending on the purposes of use (for example, preservation of the heart), the present invention may be worked without supervision of a medical doctor, if the authority and law of the particular country permit the same.

The composition or medicament of the present invention may further comprise another agent. Such an agent may be any agent known in the pharmaceutical sciences. Of course, the composition or medicament of the present invention may comprise two or more other agents. Such agents include, but are not limited to, those listed in most recent versions of the Japanese Pharmacopoeia, US Pharmacopoeia, or the pharmacopoeia of other countries. Such agents may preferably be those having effects on organs of the living organism of interest. When used during operations, agents having the same effects during operations are simultaneously administered. Such agents include, but are not limited to, for example, anti-blood clotting agents, vasodilator, tissue activator, catecholamine, PDEIII inhibitor, calcium blockers, beta blockers, steroids, and the like.

The amount of polyphenols to be administered in the composition and medicament of the present invention, may be readily determined by those skilled in the art, in consideration of the purpose of use, targeted diseases (type, severity and the like), age, weight, sex, disease history of the patient, and the course of treatment.

Preferably, the dose of polyphenols to be administered in the composition and medicament of the present invention, may be from 0.1 to 1000mg/kg/day, preferably 1-10mg/kg/day, or 10-100 mg/kg/day.

Hereinafter, the present invention is described based on examples. The following Examples are only provided for exemplary reasons. Accordingly, the scope of the present invention is not limited to the Detailed Description of the Invention or following Examples, but is only restricted by the claims.

### EXAMPLES

### EXAMPLE 1: Pre-operation effects on SD rats by oral administration

In the present Example, protective effects of a polyphenol were demonstrated using the SD rat as a model. SD rats were obtained from Shimizu Jikken Zairyo, and were treated in accordance with rules defined by Kyoto University (Japan) and were cared for in the spirit of animal protection.

SD rats were divided into two groups, namely, one receiving polyphenols and the other a control group, to which distilled water was administered. The polyphenols administered were prepared from green tea as follows: green tea was finely ground and extracted in relatively low-temperature hot water followed by isolation and purification of polyphenols using water-ethanol chromatography. Green tea polyphenols contain catechins and tannic acid as main components. Green tea polyphenols used in the present Example, contained a large amount of epigallocatechin gallate (a mixture of epigallocatechin gallate (28%), gallocatechin gallate (11.6%), epicatechin gallate (4.6 %), epigallocatechin (15%), gallocatechin (14.8%), epicatechin (7.0 %), and catechin (9.5 %)).

**TABLE 1**

| PRE-OPERATION ADMINISTRATION REGIMEN (SD RATS) | | | |
|---|---|---|---|
| Target group | Administrated sample | Dosage (ml/day) | Number of sample (n) |
| A (the present invention) | Green tea polyphenols (10⁻³ %) | 35 | 7 |
| B (control) | Distilled water | 35 | 6 |
| (Note: the administration period was fourteen days before operation) | | | |

First, each group of rats as listed in Table 1 was anesthetized with ether and pentobarbital. Thereafter, the heart was removed by median thoracotomy, and rapidly soaked in ice-cold Krebs-Henseleit Buffer (NaCl 0.6895g, KC1 0.0343g, MgSO₄ 0.145g, CaCl₂-2H₂O 0.368g, KH₂PO₄ 0.164g, NaHCO₃ 2.10g, Glucose 0.188g/100ml).

Next, the removed heart was weighed, and soon thereafter the heart was equipped with Langendorf apparatus, and perfusion was conducted using Krebs Henseleit Buffer saturated with mixed gas (oxygen 95%, carbon dioxide 5 %) for preliminary perfusion. Then, a latex balloon was connected to the left ventricle via a mistral valve from left atrium. Preliminary perfusion was performed for 20 minutes, and myocardial perfusion solution was removed in order to measure myocardium escape enzyme (GOT, GPT, CPK, CPK-MB, LDH, Toloponin-T).

The balloon was then inserted simultaneously into the left ventricle, connected to a pressure monitor via a transducer, and thereby a pressure-time relationship (dP/dt) was measured. The balloon inside the left ventricle was expanded for measuring pressure-volume relationship (ESPVR/EDPVR) thereby.

Next, 20 ml/kg of heart stopping solution cooled to 4 ° C was per fused to stop the heart. The heart stopping solution was additively administered at 10 ml/kg every 30 minutes to maintain a heart stopped state for 90 minutes in total.

Thereafter, Krebs-Henseleit Buffer was used to wash out the heart stopping solution in the heart at a flow rate of 10 ml/kg (hot shot). Thereafter, Krebs-Henseleit Buffer, saturated with mixed gas (95 % oxygen, 5 % carbon dioxide) was reperfused as a perfusion solution as in the preliminary perfusion.

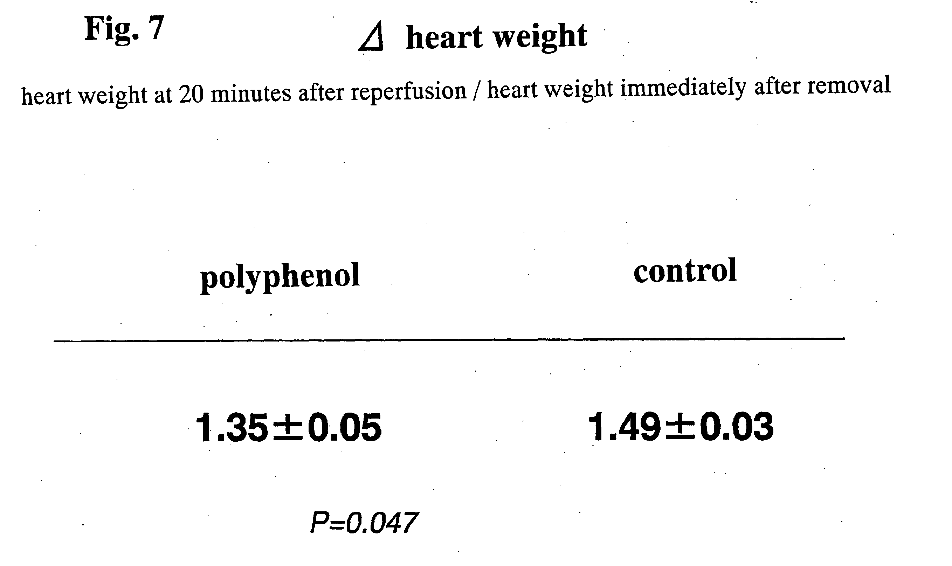

Myocardium escape enzyme and cardiac function (pressure-time relationship, heart beat number, pressure-volume relationship) were measured at 5 and 20 minutes after the start of reperfusion, and ratios against the baseline values before heart stop were calculated. Further, reperfusion was stopped 20 minutes after reperfusion, at which time the heart was weighed to calculate the weight increase ratio against the initial value (Figure 7).

At this time, the following items were removed from the target in order to precisely evaluate the effects of polyphenols:
Those showing Toloponin-T positive in perfusion determination of preliminary perfusion twenty minutes value. Abnormally high value of myocardium escape enzyme, or continually increasing value in determination after reperfusion. In addition thereto, heart beat ratio during reperfusion was 85 % or less of that of preliminary perfusion (bradycardia examples)

### (RESULTS)

### 1. Preventive effects for edema after reperfusion:

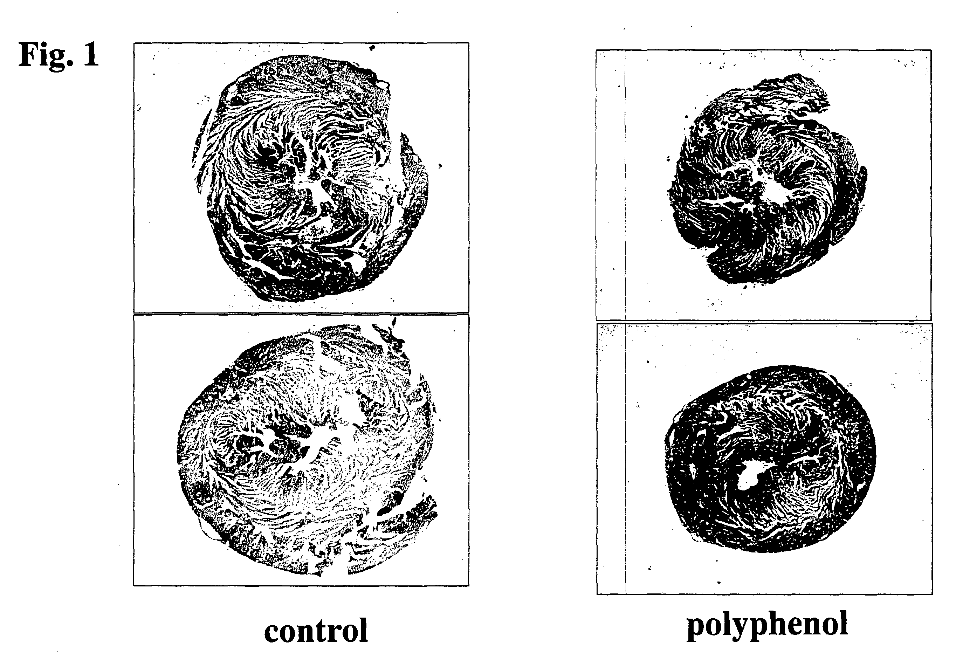

Increase in myocardium weight after reperfusion was determined: the group of preliminary administration of polyphenols showed 1.35 ± 0.05, and control groups showed 1.49 ± 0.03, which indicated significant reduction in edema (p<0.047, Figures 1 and 7).

Further, myocardium pathological specimens also showed significant suppression in left ventricle diameter and stroma edema.

### 2. Maintenance of heart systolic force:

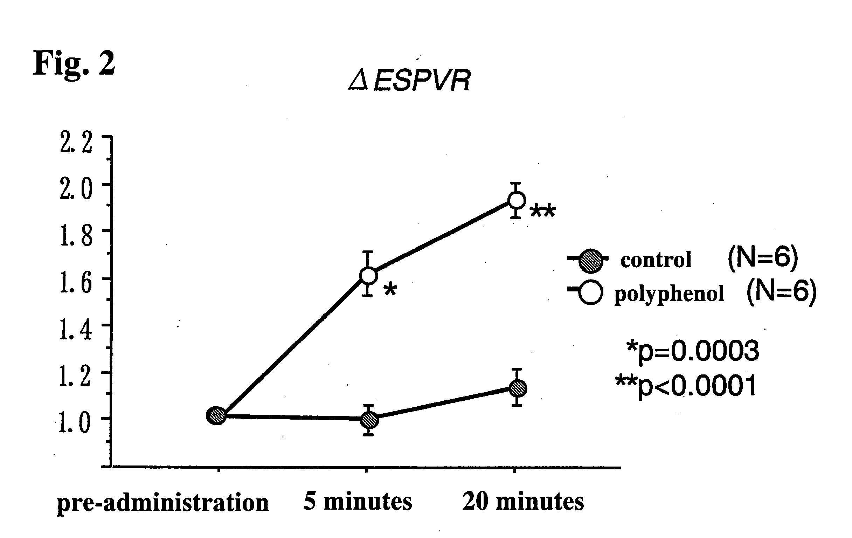

In left ventricular systolic end pressure-volume relationships, cardiac function was significantly maintained, as shown in Figure 2.

### 3. Reduction in oxidative stress

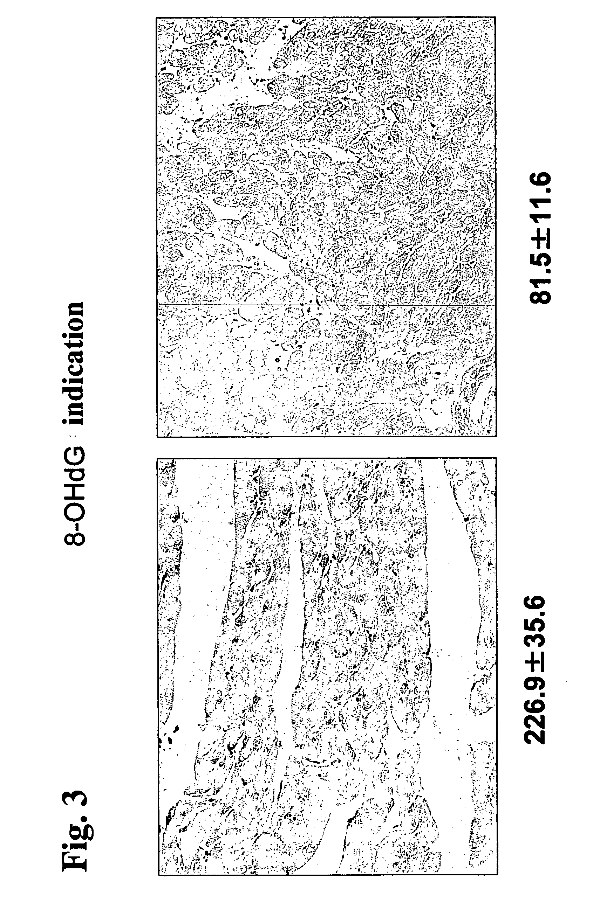

In order to determine oxidative stress, an immunopathological test using 8-OHdG, a oxidative stress marker was used, the group receiving a preliminary administration of polyphenols showed 81.5 ± 11.6, and control groups showed 226.9 ± 35.6, which indicated significant reduction in edema (p<0.0001; Figure 3).

### 4. Myocardium protection

Vacuole denaturation of the nucleus was suppressed.

As described above, according to the experiments using rats as a target, in the state of heart stop for 90 minutes, an ischemic state was maintained, and some reduction of edema was observed when measured after reperfusion. Further, according to myocardium pathological specimen, significant effects were recognized in left ventricular diameter and parenchymic stroma edema.

Heart systolic force function after reperfusion were substantially maintained as before heart stop, and it was observed that relative dysfunction was small 20 at minutes after reperfusion.

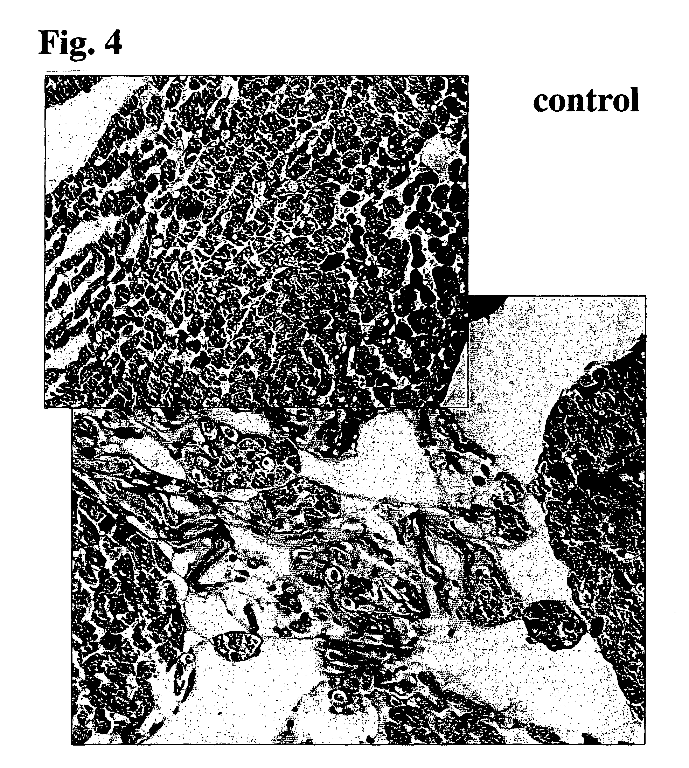

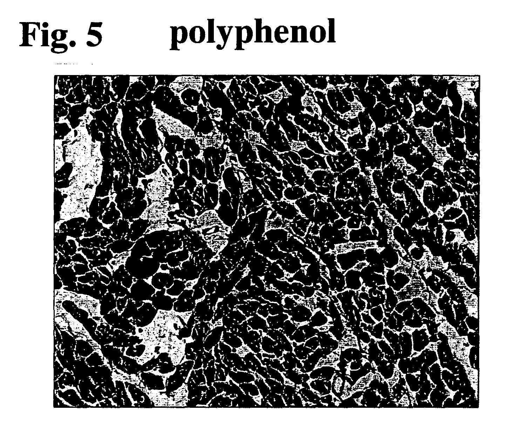

Further, as observed by histological photography (Figures 4 and 5), edema between myocardium cells was significantly reduced, and the functions were maintained at tissue and cellular levels.

Preliminary administration of polyphenols was demonstrated to be useful in three points: maintaining heart function after ischemic reperfusion, reduction of edema, and reduction in oxidative stress. Reperfusion disorder is a phenomenon which necessarily accompanies an operations using outer system circulation (extrasystem circulation). Therefore, the effects of the present invention are highly useful in a clinical setting. It is also useful in that simple administration, such as oral administration, may be used.

In conclusion, it is also possible to extrapolate for human application of the present invention. It was demonstrated that the protective function of polyphenols is generally effective in mammals in general. Such effects would not have been possible to predict in view of the prior-art. Thus, it should be noted that such protective effects brings dramatic advantages in the art.

### (EXAMPLE 2: Effects of administration effects during ischemia)

In Example 1, in lieu of pre-operation administration, administration of a tea polyphenol composition was simultaneously performed with surgery. The administration formulation was administered after disinfection.

As a result, it was revealed that prevention of edema after reperfusion was significantly attained (data not shown). Cardiac systolic force was maintained and oxidative stress was reduced, and it was observed that cardiac cells were also protected.

Further, compared with the pre-operation administration, as in Example 1, the effects of the present Example were slightly reduced.

### (EXAMPLE 3: Administration during reperfusion)

As in Example 1, in lieu of administration before operation, green tea polyphenol composition was simultaneously administered upon reperfusion. The formulation was administered after disinfection.

As a result, it was found that prevention of edema after reperfusion was significantly attained (data not shown).

Further, it was observed that heart systolic force was maintained, oxidative stress was reduced, and myocardium cells were protected. The effects observed were slightly reduced compared with the pre-operation administration of Example 1, and administration upon ischemia of Example 2. Accordingly, it is understood that pre-operation administration is more effective than administration upon ischemia or reperfusion.

### (EXAMPLE 4: Intracellular accumulation of polyphenols)

In order to study the phenomena in which polyphenols have organ protection effects before operation from a different point of view, whether or not polyphenols accumulate intracellularly was addressed.

Green tea polyphenols used in the Example 1 were FITC labeled, and oral administration using similar protocols was performed in order to study the distribution of polyphenols in the cell.

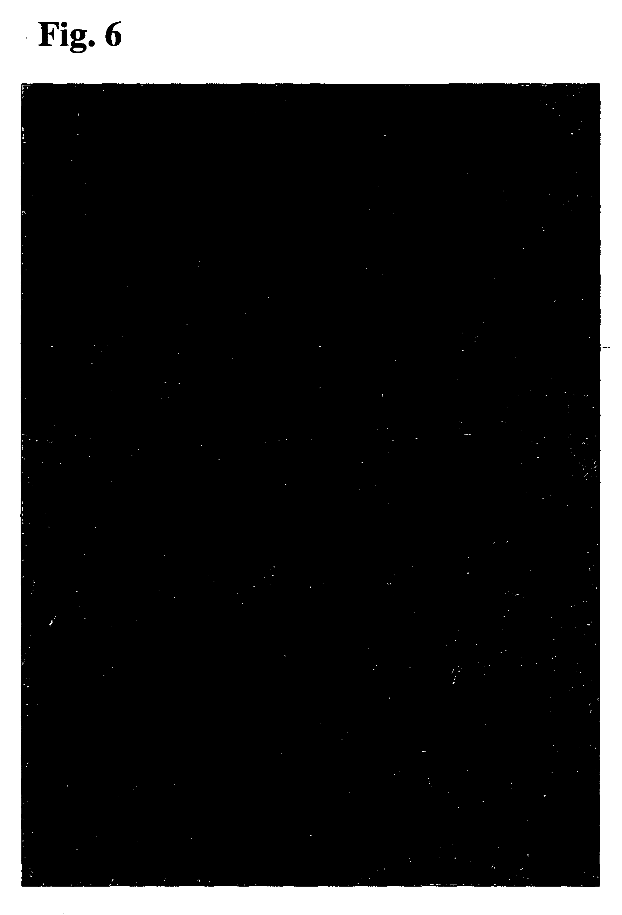

As a result, it was demonstrated that polyphenols were localized within the cellular membrane of the myocardium (Figure 6).

### (EXAMPLE 5: Effects on the brain and liver)

Next, the effects of green tea polyphenols on the brain and liver were measured as in Example 1 and similar results were obtained.

### (EXAMPLE 6: Preservative effects on the heart)

Next hearts were removed from rats (Wistar, 380 g) under anesthesia, and the preservative effects were compared and studied. As a control, hearts were cold-preserved for 48 hours after washing with UW solution. On the other hand, in the present Example, a preservation solution containing UW solution and polyphenol (10mg/ml), was used for washing kidneys upon removal, and cold-preserved for 48 hours in a similar manner. Thereafter, sustained perfusion was performed for 90 minutes using heart perfusion apparatus to measure perfusion volume, dp/dt, ESPVR for comparison. As a result, the polyphenol added system of the present invention showed significant reduction in heart disorders in comparison with the preserved kidneys using UW solution only as a comparative control.

### (EXAMPLE 7: Practice in humans)

A study was performed with human subjects to confirm the effects thereon. With respect to humans, the above-mentioned Examples were considered when determining the following protocols.

### Dosage:

800mg/day polyphenols (particularly epigallocatechin, which is believed to have potent antioxidant action) were administered via a plurality of doses, and a 1600 mg/day single dose were tested during a clinical trial (Phase 1), and the safety thereof has been confirmed. Accordingly, these dosages were herein employed. Thus, in a clinical trials for myocardium protection by the polyphenols of the present invention, similar doses such as 800-1600 mg/day were administered upon consent from patients.

### Administration period:

It was believed in light of the above animal examples, and other myocardium protection experiments and organ preservation experiments for each organ, a period of at least about two days after incorporated of polyphenols into the cell, would be required before any protective effects would be measurable in a human. Further, a regimen of daily dosage for about seven to ten days before operation is subject to a patient. However, this regimen is flexible, and may safely be extended if a patient's surgery is delayed.

### Subjects:

It should be contemplated that all treatment of cardiac vessels (such as from surgical operations to internal catheter operations and the like) will be a target for treatment.

In the present Example, treatment was applied to open heart surgery cases (cardiac vessel operations using an artificial heart lung) and off-pump by-pass operations (operations using coronal artery by-pass technology remaining autologous to the heart beat, and thus without using artificial heart lung). In this case, it was thought to preferable to administer treatment from 7 to 10 days pre-operation, and thus select non-emergency cases.

### Treatment Course

In the above-mentioned cases, no complicated diseases were observed, and it was possible that some mild headache was reported by a portion of subjects during the clinical trials, as mentioned above, however, such side effects are tolerable. However, due to property of polyphenols, it is possible that side effects relating to anemia, metabolism or excretion the like due to inhibition of iron absorption. Therefore, it is possible that side effects may be observed in the liver and kidney. However, no such effects related to the present invention were observed.

As such, it was demonstrated that the present invention is applicable to human subjects.

### (EXAMPLE 8: Seaweed extract)

Next, in lieu of tea extract, seaweed extract was used to perform the same experiments as in Examples 1-6.

Sea lettuce was obtained from commercial sources to preparing the following: sea lettuce was finely ground and extracted in relatively low-temperature hot water followed by isolation and purification of polyphenols using water-ethanol chromatography.

First, as described in Example 1, protective effects were observed for the heart when administered pre-operatively. As a result the following effects were observed, 1: prevention of edema after reperfusion; 2: maintenance of cardiac systolic action; 3: reduction of oxidative stress; and 4: protection of myocardial cell.

Next, as described in Example 2, a sea lettuce polyphenol composition was administered during ischemia. As a result, it was demonstrated that prevention of edema after reperfusion was significantly attained. Further, it was observed that cardiac systolic force was maintained, oxidative stress was reduced, and myocardial cells were protected.

Next, as described in Example 3, a sea lettuce polyphenol composition was simultaneously administered during reperfusion. As a result, it was demonstrated that prevention of edema after reperfusion was significantly attained. Further, it was observed that cardiac systolic force was maintained, oxidative stress was reduced, and myocardium cell were protected.

Accumulation of sea lettuce polyphenols in the body was addressed as in Example 4, and it was observed that the distribution of polyphenol of myocardium is localized within the cellular membrane.

As such, it was observed that polyphenols from sea lettuce attain similar results to those of tea extract.

Further, as described in Example 5, experiments in the brain and liver were performed, and the same results were obtained. As described in Example 6, extracorporeal preservation effects on the heart were observed and preservation effects thereof were found.

### (EXAMPLE 9: Aloe extract)

Next, in lieu of tea extract, aloe extract was used to perform the same experiments as in Examples 1-6.

Aloe vera was obtained from commercial source for preparing the following: aloes were finely ground and extracted in relatively low-temperature hot water followed by isolation and purification of polyphenols using water-ethanol chromatography.

First, as described in Example 1, protective effects were observed for the heart when administered pre-operatively. As a result the following effects were observed, 1: prevention of edema after reperfusion; 2: maintenance of cardiac systolic action; 3: reduction of oxidative stress; and 4: protection of myocardial cell.

Next, as described in Example 2, an aloe polyphenol composition was administered during ischemia. As a result, it was demonstrated that prevention of edema after reperfusion was significantly attained. Further, it was observed that cardiac systolic force was maintained, oxidative stress was reduced, and myocardial cells were protected.

Next, as described in Example 3, an aloe polyphenol composition was simultaneously administered during reperfusion. As a result, it was demonstrated that prevention of edema after reperfusion was significantly attained. Further, it was observed that cardiac systolic force was maintained, oxidative stress was reduced, and myocardium cell were protected.

Accumulation of aloe polyphenol in the body was observed as in Example 4, and it was observed that the distribution of polyphenol of myocardium is localized within the cellular membrane.

As such, it was observed that polyphenols from aloe attain similar results to those of tea extract.

Further, as described in Example 5, experiments in the brain and liver were performed, and the same results were obtained. As described in Example 6, extracorporeal preservation effects on the heart were observed and preservation effects thereof were found.

### (EXAMPLE 10: wine extract)

Next, in lieu of tea extract, red wine extract was used to perform the same experiments as in Examples 1-6.

Red wine (from France, Bordeaux, 2000; commercially available) was obtained from a commercial source to prepare the following: red wine was microfluidized and extracted in relatively low-temperature hot water to isolate and purify polyphenols using water-ethanol chromatography. Red wine polyphenols contain flavonol, catechins as main components.

First, as described in Example 1, protective effects were observed for the heart when administered pre-operatively. As a result the following effects were observed, 1: prevention of edema after reperfusion; 2: maintenance of cardiac systolic action; 3: reduction of oxidative stress; and 4: protection of myocardial cell.

Next, as described in Example 2, a red wine polyphenol composition was administered during ischemia. As a result, it was demonstrated that prevention of edema after reperfusion was significantly attained. Further, it was observed that cardiac systolic force was maintained, oxidative stress was reduced, and myocardial cells were protected.

Next, as described in Example 3, a red wine polyphenol composition was simultaneously administered during reperfusion. As a result, it was demonstrated that prevention of edema after reperfusion was significantly attained. Further, it was observed that cardiac systolic force was maintained, oxidative stress was reduced, and myocardium cell were protected.

Accumulation of red wine polyphenols in the body was observed as in Example 4, and it was observed that the distribution of polyphenols in the myocardium is localized within the cellular membrane.

As such, it was observed that polyphenols from red wine attain similar results to those of tea extract.

Further, as described in Example 5, experiments in the brain and liver were performed, and the same results were obtained. As described in Example 6, extracorporeal preservation effects on the heart were observed and preservation effects thereof were found.

### (EXAMPLE 11: Cactus extract)

Next, in lieu of tea extract, cactus extract was used to perform the same experiments as in Examples 1-6.

Cacti were obtained from a commercial source to prepare the following: cacti were finely ground and extracted in relatively low-temperature hot water to isolate and purify polyphenols using water-ethanol chromatography.

First, as described in Example 1, protective effects were observed for the heart when administered pre-operatively. As a result the following effects were observed, 1: prevention of edema after reperfusion; 2: maintenance of cardiac systolic action; 3: reduction of oxidation stress; and 4: protection of myocardial cell.

Next, as described in Example 2, a cactus polyphenol composition was administered during ischemia. As a result, it was demonstrated that prevention of edema after reperfusion was significantly attained. Further, it was observed that cardiac systolic force was maintained, oxidative stress was reduced, and myocardial cells were protected.

Next, as described in Example 3, a cactus polyphenol composition was simultaneously administered during reperfusion. As a result, it was demonstrated that prevention of edema after reperfusion was significantly attained. Further, it was observed that cardiac systolic force was maintained, oxidative stress was reduced, and myocardium cell were protected.

Accumulation of cactus polyphenol in the body was observed as in Example 4, and it was observed that the distribution of polyphenols in the myocardium was localized within the cellular membrane.

As such, it was observed that polyphenols from cacti attain similar results to those of tea extract.

Further, as described in Example 5, experiments in the brain and liver were performed, and the same results were obtained. As described in Example 6, extracorporeal preservation effects on the heart were observed and preservation effects thereof were found.

### (EXAMPLE 11: Fruit extract)

Next, in lieu of tea extract, fruit extract was used to perform the same experiments as in Examples 1-6.

Grapes were obtained from a commercial source to preparing the following: fruits were finely ground and extracted in relatively low-temperature hot water to isolate and purify polyphenols using water-ethanol chromatography.

First, as described in Example 1, protective effects were observed for the heart when administered pre-operatively. As a result the following effects were observed, 1: prevention of edema after reperfusion; 2: maintenance of cardiac systolic action; 3: reduction of oxidative stress; and 4: protection of myocardial cell.

Next, as described in Example 2, a fruit polyphenol composition was administered during ischemic period. As a result, it is demonstrated that prevention of edema after reperfusion is significant attained. Further, it is observed that cardiac systolic force is maintained, oxidative stress is reduced, and myocardium cell is protected.

Next, as described in Example 3, a fruit polyphenol composition was simultaneously administered during reperfusion. As a result, it was demonstrated that prevention of edema after reperfusion was significantly attained. Further, it was observed that cardiac systolic force was maintained, oxidative stress was reduced, and myocardium cell were protected.

Accumulation of fruit polyphenols in the body was observed as in Example 4, and it was observed that the distribution of polyphenols in the myocardium was localized within the cellular membrane.

As such, it was observed that polyphenols from fruits attain similar results to those of tea extract.

Further, as described in Example 5, experiments in the brain and liver were performed, and the same results were obtained. As described in Example 6, extracorporeal preservation effects on the heart were observed and preservation effects thereof were found.

Although certain preferred embodiments have been described herein, it is not intended that such embodiments be construed as limitations on the scope of the invention except as set forth in the appended claims. Various other modifications and equivalents will be apparent to and can be readily made by those skilled in the art, after reading the description herein, without departing from the scope and spirit of this invention. All patents, published patent applications and publications cited herein are incorporated by reference as if set forth fully herein.

### INDUSTRIAL APPLICABILITY

The protection and preservation effects of the polyphenols of the present invention on organs, tissues or cells has been confirmed. Such effects may be applicable during operations and the like, and used as a pharmaceutical composition. In particular, the present invention is highly useful for enhancing flexibility during operations.

## Claims

1. A composition for protecting and preserving an organ, a tissue, or a cell, comprising a polyphenol.

2. The composition according to Claim 1, wherein the polyphenol is present in an amount sufficient for preservation of the organ, tissue or cell during ischemia or reperfusion.

3. The composition according to Claim 2, wherein the ischemia and reperfusion arises in surgery or medical operation.

4. The composition according to Claim 1, wherein the preservation is for operation, and the polyphenol is administered during the time from pre-operation to post-reperfusion.

5. The composition according to Claim 4, wherein the administration is administered at a time from at least two weeks before an operation to the date of the operation.

6. The composition according to Claim 4, wherein the administration is administered at least at the time of ischemia.

7. The composition according to Claim 4, wherein the administration is performed at least on reperfusion.

8. The composition according to Claim 4, wherein the administration is performed at least after reperfusion.

9. The composition according to Claim 4, wherein the administration is performed orally or parenterally.

10. The composition according to Claim 4, wherein the operation is surgical or internally.

11. The composition according to Claim 10, wherein the operation is surgical.

12. The composition according to Claim 11, wherein the surgical operation uses off-pump, PCI, catheter intervention or extracorporeal circulation.

13. The composition according to Claim 11, wherein the surgical operation is an operation of aorta, arteries coronaria or valve.

14. The composition according to Claim 1, wherein the organ, tissue or cell is an organ.

15. The composition according to Claim 1, wherein the organ comprises the skin, blood vessels, cornea, kidney, heart, liver, umbilical cord, intestine, nerve, lung, placenta, or pancreas.

16. The composition according to Claim 1, wherein the organ is selected from the group consisting of the heart, brain, nerve and spinal cord.

17. The composition according to Claim 1, wherein the organ, tissue or cell is mammalian.

18. The composition according to Claim 1, wherein the organ, tissue or cell is human.

19. The composition according to Claim 1, which is a protective agent for the organ, tissue or cell.

20. The composition according to Claim 19, wherein the protection comprises protection in ischemia.

21. The composition according to Claim 1, which is a preservation agent for the organ, tissue or cell.

22. The composition according to Claim 1, wherein the polyphenol is a mixture or a single component.

23. The composition according to Claim 1,wherein the polyphenol is a catechin, tannin, proanthocyanidine, or resveratrol.

24. The composition according to Claim 1, wherein the polyphenol comprises at least catechins or tannins.

25. The composition according to Claim 1, wherein the polyphenol comprises epigallocatechingallate.

26. The composition according to Claim 1, wherein the polyphenol has a hydroxy value of about 2 to about 100.

27. The composition according to Claim 1, wherein the polyphenol comprises an extract selected from the group consisting of tea extract, seaweed extract, aloe extract, wine extract, cactus extract and fruit extract.

28. The composition according to Claim 1 wherein the polyphenol comprises seaweed extract.

29. The composition according to Claim 1, wherein the polyphenol comprises tea extract.

30. A method for protecting an organ, tissue or cell in a subject, comprising:
1) exposing the organ, tissue or cell to a polyphenol.

31. The method according to Claim. 30, wherein the polyphenol is administered to the subject in an amount effective for protecting the organ, tissue or cell, during ischemia or reperfusion.

32. The method according to Claim 30, wherein the protection is performed at operation.

33. The method according to Claim 30, wherein the operation is surgical or internal.

34. The method according to Claim 32, wherein the polyphenol is administered to the subject at any period of time from pre-operation to reperfusion.

35. The method according to Claim 34, wherein the administration is performed at any time point from at least two weeks before operation to the date of operation.

36. The method according to Claim 34, wherein the administration is performed at least at ischemia.

37. The method according to Claim 34, wherein the administration is performed at least at reperfusion.

38. The method according to Claim 34, wherein the administration is performed at least after reperfusion.

39. The method according to Claim 34, wherein the administration is performed orally or parenterally.

40. The method according to Claim 32, wherein the operation is surgical.

41. The method according to Claim 32, wherein the surgical operation uses off-pump, PCI, catheter intervention or extracorporeal circulation.

42. The method according to Claim 40, wherein the surgery operation is an operation on the aorta, arteria coronaria or valve.

43. The method according to Claim 30, wherein the organ, tissue or cell is an organ.

44. The method according to Claim 30, wherein the organ comprises the skin, blood vessels, cornea, kidney, heart, liver, umbilical cord, intestine, nerve, lung, placenta, or pancreas.

45. The method according to Claim 30, wherein the organ is selected from the group consisting of the heart, brain, nerve and spinal cord.

46. The method according to Claim 30, wherein the organ, tissue or cell is mammalian.

47. The method according to Claim 30, wherein the organ, tissue or cell is human.

48. The method according to Claim 30, wherein the protection comprises protection during ischemia.

49. The method according to Claim 30, wherein the protection comprises protection at reperfusion.

50. The method according to Claim 30, wherein the polyphenol is a mixture or a single component.

51. The method according to Claim 30, wherein the polyphenol is a catechin, tannin, proanthocyanidine, or resveratrol.

52. The method according to Claim 30, wherein the polyphenol comprises at least catechins or tannins.

53. The method according to Claim 30, wherein the polyphenol comprises epigallocatechingallate.

54. The method according to Claim 30, wherein the polyphenol has a hydroxy value of about 2 to about 100.

55. The method according to Claim 30, wherein the polyphenol comprises an extract selected from the group consisting of tea extract, seaweed extract, aloe extract, wine extract, cactus extract and fruit extract.

56. The method according to Claim 30, wherein the polyphenol comprises seaweed extract.

57. The method according to Claim 30, wherein the polyphenol comprises tea extract.

58. A method for preserving an organ, tissue or cell, comprising the step of:
1) retaining the organ, tissue or cell in a fluid comprising a polyphenol.

59. The method according to Claim 58, wherein the organ is the heart.

60. Use of polyphenol in manufacturing a medicament for protecting or preserving an organ, tissue or cell, wherein the medicament comprises the polyphenol.
